# FASCICULE DE BREVET EUROPEEN

(11) **EP 1 105 456 B1**
(45) Date de publication et mention de la délivrance du brevet: **12.11.2003**
(21) Numéro de dépôt: 99936736.0
(22) Date de dépôt: 16.08.1999
(51) Int. Cl.: C12M 1/00, A23K 1/165

(54) **DISPOSITIF DE MESURE RAPIDE DE L'ACTIVITE ENZYMATIQUE**
VORRICHTUNG ZUR SCHNELLEN MESSUNG DER AKTIVITÄT VON ENZYMEN
FAST MEASURING DEVICE OF ENZYMATIC ACTIVITY

(30) Priorité: 19.08.1998 FR 9810533
(43) Date de publication de la demande: 13.06.2001
(73) Titulaire: AVENTIS ANIMAL NUTRITION S.A., 92160 Antony (FR)
(72) Inventeur: ROBERTS, Neil, Stockport, Cheschire SK6 1EF (GB); MOORES, Janet, Stockport, Cheschire SK6 4AP (GB)
(74) Mandataire: Guerre, Dominique
(86) Numéro de dépôt international: FR9901990
(87) Numéro de publication internationale: WO00011136

(56) Documents cités:
- EP-A- 0 223 745
- EP-A- 0 381 173
- EP-A- 0 493 758
- US-A- 4 277 561

## Description

La présente invention concerne un dispositif de mesure rapide d'une activité enzymatique dans un aliment solide comprenant (i) un récipient destiné à contenir l'échantillon à tester, (ii) un réactif propre à l'enzyme dont on veut mesurer l'activité et (iii) un tampon permettant la mise en solution de l'enzyme.

L'aliment est de préférence un aliment solide et non traité préalablement à la mesure.

Les aliments destinés aux animaux d'élevage sont usuellement additionnés d'enzymes dont le rôle est principalement une meilleure digestibilité de la ration alimentaire. Ces enzymes sont le plus souvent pulvérisées sous forme liquide sur les aliments, tel que notamment décrit dans le brevet EP 0789291. Les enzymes peuvent aussi être additionnées sous forme de poudre dans l'aliment.

Deux problèmes se posent alors, le premier est de vérifier l'uniformité de la répartition des enzymes ajoutées à l'aliment, le second est d'évaluer de façon rapide et facile l'activité de la ou des enzymes ajoutées aux aliments. Ces problèmes sont en particulier soulevés par les fabricants d'aliments et les éleveurs voulant vérifier la qualité des aliments qu'ils veulent donner à leurs animaux. Jusqu'à présent, l'activité enzymatique pouvait être mesurée en laboratoire, ce qui entraînaient des contraintes en terme de logistique et de délais, ces contraintes étaient gênantes en cas de besoin d'un résultat immédiat. Parmi les mesures qui peuvent être réalisées, le document US-A-4,277,561 décrit un dispositif et un procédé pour mesurer l'activité enzymatique d'un extrait biologique. Le dispositif comprend un support qui est fait d'un matériel fibreux imprégné avec un substrat enzymatique et un procédé stabilisateur de pH. L'activité enzymatique de l'extrait est mesurée grâce à un procédé colorimétrique en déposant ledit extrait sur le support et en le laissant réagir avec un réactif comprenant un chromophore dissous dans un tampon. Ce type de dispositif n'est pas adapté pour la mesure d'une activité enzymatique à partir d'un échantillon solide.

La présente invention répond à ce problème en proposant un dispositif de mesure de l'activité enzymatique de n'importe quel aliment enrichi en enzyme destiné à l'alimentation animale. Ce dispositif, dont la mesure repose sur une réaction colorimétrique, permet à la fois une mesure qualitative de l'activité enzymatique de l'échantillon testé et une mesure semi-quantitative de cet échantillon.

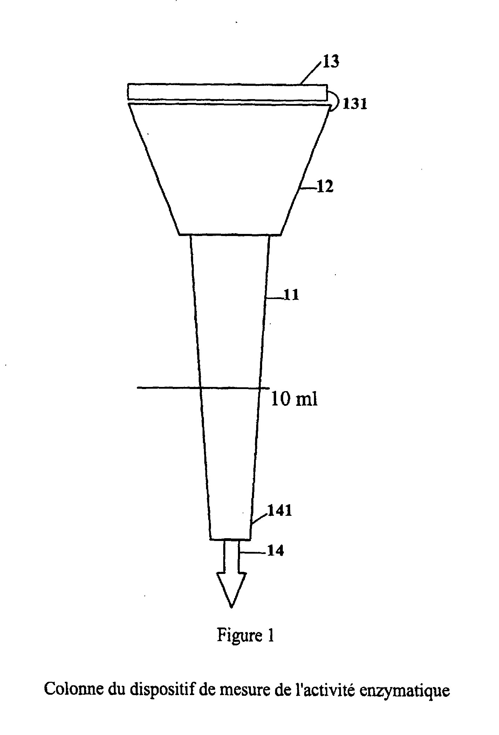

La figure 1 représente un moyen de mise en oeuvre de l'invention sous forme d'un dispositif de mesure de l'activité enzymatique se présentant sous la forme d'une colonne..

La description suivante peut se lire au regard de la figure ci-dessus mentionnée.

Le dispositif objet de la présente invention comprend un récipient destiné à contenir l'échantillon à tester, un réactif propre à l'enzyme dont on veut mesurer l'activité et un tampon permettant la mise en solution de ladite enzyme.

Le récipient de ce dispositif peut être, de façon non limitative, une colonne (figure 1) composée d'une partie inférieure étroite graduée (11) et d'une partie supérieure large en forme d'entonnoir (12) facilitant l'introduction des différents réactifs dans la colonne ainsi que leur mélange lors de l'agitation. La colonne est munie d'un système d'ouverture et de fermeture étanche (13) telle qu'un bouchon relié au corps de la colonne par une languette (131).

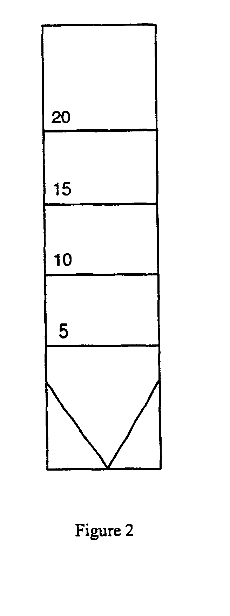

Le récipient peut aussi être constitué d'un tube à usage unique (figure 2).

Le récipient peut être en matière synthétique telle qu'un plastique à usage unique.

De façon préférée, le récipient peut comporter une excroissance sécable (14) à sa base permettant l'écoulement de la partie liquide de son contenu. L'excroissance est avantageusement surmontée d'un étranglement (141) retenant les morceaux d'aliments solides.

La mesure de l'activité enzymatique est basée sur la réaction de coloration de la méthode Azo. Le principe de la réaction de coloration de la méthode Azo repose sur l'hydrolyse enzymatique d'un substrat caractéristique d'une enzyme lié à un chromophore. La réaction produit des oligomères solubles qui colorent en bleu le milieu. L'absorbance du milieu peut être mesurée à 590 nm.

Le réactif utilisé dans le dispositif est le substrat de la réaction catalysée par l'enzyme lié à un chromophore. Ainsi, la réaction d'hydrolyse enzymatique libère le substrat chromophore.

Le dispositif comprend également un tampon permettant la mise en solution des enzymes qui ont été pulvérisées sur l'aliment ainsi que le maintien des enzymes à leur pH optimum.

On peut citer, à titre d'exemple et de façon non restrictive, le dispositif de mise en évidence de l'activité des xylanases.

Pour la mesure de l'activité des xylanases, le réactif utilisé est l'''Oat spelt Xylan Remazol Brillant Blue R" ou le "Xylazyme AX" (vendus par la société Megazyme et constitué d'araboxylane d'avoine ou de blé lié à un colorant ).

Le tampon utilisé est choisi parmi les tampons acide acétique/acétate de sodium; chlorhydrate de glycine/glycine; acide aconitique/hydroxyde de sodium; acide formique/formate de sodium.

On peut encore citer le dispositif de mise en évidence de l'activité des β-glucanases qui repose également sur la réaction de coloration de la méthode Azo.

Parmi les substrats utilisables on peut citer le 1,3 : 1,4-β-D-glucan avec Remazol Brillant Blue R et le Beta-Glucazyme vendu par la société Megazyme et constitué de béta glucane associé à de l'Azurine.

Le tampon utilisé est choisi parmi les tampons acide acétique/acétate de sodium; chlorhydrate de glycine/glycine; acide aconitique/hydroxyde de sodium; acide formique/formate de sodium.

Pour la mesure de l'activité de la cellulase, le substrat utilisé est sous forme de tablettes Cellazyme (commercialisée par la société Megazyme). Ces tablettes sont constituées de substrats à base de cellulose et ou de cellulose et de xyloglucanes polymérisés avec un colorant azurine.

Dans un mode préféré de réalisation de la présente invention, le réactif se présente sous une forme solide.

Avantageusement, pour faciliter la mise en solution de l'enzyme on peut ajouter au substrat contenant l'agent chromophore un tensioactif. Ce tensioactif est choisi notamment parmi le laurylsulfate de sodium ou le dodécylsulfate de sodium.
Selon un meilleur moyen de mise en oeuvre de l'invention la mesure est réalisé en quatre étapes :
- introduction dans le récipeint (1) de 10 ml d'échantillon dont on veut mesurer l'activité enzymatique, pour un échantillon solide, il faut remplir de solide jusqu'à la graduation 10 ml ;
- introduction du réactif sous la forme d'une bille solide ;
- introduction du tampon spécifique jusqu'à la graduation 20 ml ;
- après fermeture de la colonne avec le bouchon, cette dernière est agitée vigoureusement à plusieurs reprises ;

On peut éventuellement ajouter une étape supplémentaire de séparation de la phase liquide et de la phase solide (par centrifugation ou filtration) pour récupérer la phase liquide et mesurer l'intensité de la coloration par spectrophotométrie ou simplement par comparaison avec une échelle colorée..

L'apparition d'une coloration bleue après un temps de réaction de 4 à 8 heures confirme la présence d'enzymes actives, l'intensité de la coloration est proportionnelle à l'activité des enzymes présentes dans l'échantillon.

Un autre avantage de la présente invention est de pouvoir effectuer une mesure semi-quantitative de l'activité enzymatique. La phase liquide colorée de la colonne peut être récupérée par coupure de l'excroissance sécable de la colonne. L'intensité de sa coloration peut alors être comparée avec une courbe d'étalonnage de DO.

En plus d'être rapide, la méthode de mesure est très simple et le dispositif peut être utilisé n'importe où sans nécessiter d'équipement spécial. Par exemple, un fabricant ou un éleveur peut faire une mesure de contrôle dès la fabrication de l'aliment.

La présente invention sera plus compètement décrite à l'aide des exemples suivants qui ne doivent pas être considérés comme limitatifs de l'invention.

### Exemples

Deux séries d'essais ont été menées sur du Rovabio xylan LC (mélange de xylanase et béta glucanase de *Pénicillium funiculosum*) et sur du Rovabio xylanase TRLC (xylanase de *Trichoderma reesei*) dont l'activité xylanase est comprise entre.350 et 550 uAXC /ml. On estime que le traitement de pulvérisation de la composition liquide sur les aliments conduit à un taux de 70 à 110 uAXC/kg d'aliment.

Le tampon utilisé est le tampon acétate maintenant un pH à 4.7. La pulvérisation peut être effectuée sur l'aliment pulvérulent ou sous forme granulé.

| **échantillon** | Activité (avant ajustement) | observation à 3 heures | D.O. à 590 nm à 4h30 | D.O. à 590 nm à 8h | observation à 8h |
|---|---|---|---|---|---|
| xylanase TRLC sur granulés | 1336 | bleu : +++ | >3,0 | >3,0 | bleu: +++ |
| xylanase TRLC sur granulés | 886,7 | bleu : + | 1,567 | 2,685 | bleu : ++ |
| xylanase TRLC sur granulés | 1469,25 | bleu : + | 1,429 | 2,652 | bleu : ++ |
| xylanase poudre avant granulation | 631,4 | pas de coloration | 0,201 | 0,666 | bleu: + |
| xylanase LC sur granulés | 1144 | bleu : +++ | 2,309 | 2,376 | bleu : +++ |
| xylanase LC sur granulés | 1386,7 | bleu : + | 1,382 | 2,484 | bleu : +++ |
| xylanase LC sur granulés | 1450,5 | bleu : +++ | 2,872 | 2,85 | bleu : +++ |
| xylanase LC sur granulés | 1330,5 | bleu : ++ | 1,233 | 2,096 | bleu : +++ |

## Revendications

1. Dispositif de mesure d'activité enzymatique d'un échantillon alimentaire solide **caractérisé en ce qu'**il comprend un récipient destiné à contenir l'échantillon à tester, un réactif propre à l'enzyme dont on veut mesurer l'activité et un tampon permettant la mise en solution de ladite enzyme et **en ce que** ledit récipient est une colonne composée d'une partie inférieure étroite graduée et d'une partie supérieure large en forme d'entonnoir facilitant l'introduction des différents réactifs dans la colonne ainsi que leur mélange lors de l'agitation, ladite colonne étant munie d'un système d'ouverture et de fermeture étanche.

2. Dispositif selon la revendication 1, **caractérisé en ce que** l'échantillon à tester est un aliment solide de préférence non traité.

3. Dispositif selon la revendication 1 ou 2, **caractérisé en ce que** ledit récipient est une colonne graduée à usage unique.

4. Dispositif selon la revendication 3, **caractérisé en ce que** ledit récipient comporte une excroissance sécable à sa base permettant l'écoulement de la partie liquide de son contenu.

5. Dispositif selon l'une quelconque des revendications 1 à 4, **caractérisé en ce que** le réactif est le substrat de l'enzyme lié à un chromophore.

6. Dispositif selon l'une quelconque des revendications 1 à 5, **caractérisé en ce que** le réactif se présente sous forme solide ou liquide.

7. Dispositif selon la revendication 1, **caractérisé en ce que** le tampon utilisé pour mesurer l'activité de l'enzyme est choisi parmi les tampons acide acétique/acétate de sodium ; chlorhydrate de glycine/glycine ; acide aconitique/hydroxyde de sodium ; acide formique/formate de sodium.

8. Utilisation du dispositif selon la revendication 1 pour mesurer l'activité enzymatique quantitativement **caractérisé en ce qu'**on compare la coloration obtenue avec une courbe étalon.

9. Procédé de mesure de l'activité enzymatique d'un aliment **caractérisé en ce que** on introduit dans le dispositif selon les revendications 1 à 5, 10 ml d'échantillon dont on veut mesurer l'activité enzymatique, on introduit du réactif sous la forme d'une bille solide ; on introduit du tampon spécifique jusqu'à la graduation 20 ml ; après fermeture de la colonne avec le bouchon, cette dernière est agitée vigoureusement à plusieurs reprises ; on sépare la phase liquide de la phase solide, on récupère la phase liquide et on mesurer l'intensité de la coloration par comparaison avec une échelle colorée.

## Claims

1. Device for measuring the enzymatic activity of a solid feed sample, **characterized in that** it comprises a container designed to contain the test sample, a reagent specific for the enzyme whose activity it is desired to measure and a buffer for dissolving the said enzyme, and **in that** the said container is a column composed of a graduated narrow bottom part and a wide funnel-shaped top part that facilitates the introduction of the various reagents into the column and also their mixing during stirring, the said column being fitted with a leakproof opening and closure system.

2. Device according to Claim 1, **characterized in that** the test sample is a solid feed, which is preferably untreated.

3. Device according to Claim 1 or 2, **characterized in that** the said container is a single-use graduated column.

4. Device according to Claim 3, **characterized in that** the said container comprises a cleavable protuberance at its base, allowing the liquid part of its contents to flow out.

5. Device according to any one of Claims 1 to 4, **characterized in that** the reagent is the substrate for the enzyme linked to a chromophore.

6. Device according to any one of Claims 1 to 5, **characterized in that** the reagent is in solid or liquid form.

7. Device according to Claim 1, **characterized in that** the buffer used to measure the activity of the enzyme is chosen from acetic acid/sodium acetate; glycine hydrochloride/glycine; aconitic acid/sodium hydroxide; formic acid/sodium formate buffers.

8. Use of the device according to Claim 1, to measure enzymatic activity quantitatively, **characterized in that** the coloration obtained is compared with a standard curve.

9. Process for measuring the enzymatic activity of a feed, **characterized in that** 10 ml of sample whose enzymatic activity it is desired to measure are introduced into the device according to Claims 1 to 5, reagent in the form of a solid bead is introduced; specific buffer is introduced up to the 20 ml graduation mark; after closure of the column with the stopper, the column is shaken vigorously several times; the liquid phase is separated from the solid phase, the liquid phase is recovered and the intensity of the coloration is measured by comparison with a colour scale.

## Patentansprüche

1. Vorrichtung zur Messung von enzymatischer Aktivität einer festen Nahrungsmittelprobe, **dadurch gekennzeichnet, dass** die Vorrichtung ein Behältnis, das dazu bestimmt ist, die zu testende Probe zu enthalten, ein Reagenz, das für das Enzym geeignet ist, dessen Aktivität zu messen ist, und einen Puffer, der das In-Lösung-bringen des Enzymes ermöglicht, aufweist, und dass das Behältnis eine Säule ist, die aus einem unteren engen mit einer Skalierung versehenen Teil und aus einem breiten oberen Teil unter Ausbildung eines Trichters zusammengesetzt ist, wodurch das Einbringen von verschiedenen Reagenzien in die Säule sowie deren Mischung bei Bewegung erleichtert wird, wobei die Säule mit einem System zum Öffnen und zum dichten Verschließen versehen ist.

2. Vorrichtung nach Anspruch 1, **dadurch gekennzeichnet, dass** die zu testende Probe ein festes, vorzugsweise unbehandeltes Nahrungsmittel ist.

3. Vorrichtung nach Anspruch 1 oder 2, **dadurch gekennzeichnet, dass** das Behältnis eine mit einer Skalierung versehene Säule zur einmaligen Verwendung ist.

4. Vorrichtung nach Anspruch 3, **dadurch gekennzeichnet, dass** das Behältnis an seiner Basis eine schneidbare Ausstülpung aufweist, die den Abfluss des flüssigen Teils von dessen Inhalt ermöglicht.

5. Vorrichtung nach einem der Ansprüche 1 bis 4, **dadurch gekennzeichnet, dass** das Reagenz das an ein Chromophor gebundene Substrat des Enzymes ist.

6. Vorrichtung nach einem der Ansprüche 1 bis 5, **dadurch gekennzeichnet, dass** das Reagenz in fester oder flüssiger Form vorliegt.

7. Vorrichtung nach Anspruch 1, **dadurch gekennzeichnet, dass** der für die Messung der Enzymaktivität verwendete Puffer ausgewählt ist aus den Essigsäure/Natriumacetat-; Glycin-Chlorhydrat/Glycin-; Aconitsäure/Natriumhydroxyd-; Ameisensäure/Natriumformiat-Puffern.

8. Verwendung der Vorrichtung gemäß Anspruch 1, um die enzymatische Aktivität quantitativ zu messen, **dadurch gekennzeichnet, dass** die erhaltene Färbung mit einer Eichkurve verglichen wird.

9. Verfahren, um die enzymatische Aktivität eines Nahrungsmittels zu messen, **dadurch gekennzeichnet, dass** 10 ml einer Probe, deren enzymatische Aktivität zu messen ist, und ein Reagenz in Form einer festen Kugel in die Vorrichtung gemäß der Ansprüche 1 bis 5 eingebracht werden; ein spezifischer Puffer bis zur Skalierung 20 ml eingebracht wird; nach Verschließen der Säule mit dem Verschluss die Säule mehrfach wiederholend kräftig geschüttelt wird; die flüssige Phase von der festen Phase getrennt wird, die flüssige Phase zurückgewonnen und die Intensität der Färbung durch den Vergleich mit einer farbigen Skala gemessen werden.
